# EUROPEAN PATENT SPECIFICATION

(11) **EP 3 570 027 B1**
(45) Date of publication and mention of the grant of the patent: **20.12.2023**
(21) Application number: 17903001.0
(22) Date of filing: 30.03.2017
(51) Int. Cl.: G01N 33/497, A61B 5/08, A61B 5/097

(54) **SULFIDE GAS CONCENTRATION MEASUREMENT DEVICE AND SULFIDE GAS CONCENTRATION MEASUREMENT METHOD**
VORRICHTUNG UND VERFAHREN ZUR MESSUNG DER KONZENTRATION VON SCHWEFELWASSERSTOFFGAS
DISPOSITIF DE MESURE DE CONCENTRATION DE GAZ DE SULFURE ET PROCÉDÉ DE MESURE DE CONCENTRATION DE GAZ DE SULFURE

(43) Date of publication of application: 20.11.2019
(73) Proprietor: JMS Inc., Tokyo 140-0011 (JP); Saito, Junpei, Fukushima-shi, Fukushima 960-8157 (JP)
(72) Inventor: SAITO, Junpei, Fukushima-shi Fukushima 960-8157 (JP); HIGASHIDE, Kazusa, Tokyo 140-0011 (JP); KAWAMOTO, Ken, Tokyo 140-0011 (JP); YAMAMOTO, Yasumichi, Tokyo 140-0011 (JP)
(74) Representative: Henkel & Partner mbB
(86) International application number: PCT/JP2017/013137
(87) International publication number: WO 2018/179195

(56) References cited:
- WO-A1-2016/200948
- WO-A1-2017/040546
- JP-A- 2000 506 601
- JP-A- 2005 538 819
- JP-A- 2010 217 016
- JP-A- 2014 522 973
- US-A1- 2014 305 810
- US-A1- 2015 032 019
- US-A1- 2017 065 208
- ZHANG, J. et al.: "Exhaled Hydrogen Sulfide Predicts Airway Inflammation Phenotype in COPD", RESPIRATORY CARE, vol. 60, no. 2, 2015, pages 251-258, XP055550555,

## Description

### Technical Field

The present invention relates to a sulfide gas measuring device and a sulfide gas measuring method using the sulfide gas measuring device, more particularly to a sulfide gas measuring device and a sulfide gas measuring method suitable for measuring the concentration of sulfide gas contained in exhaled breath.

### Background Art

In recent years, the concentration of sulfide gas (e.g., hydrogen sulfide) contained in exhaled breath has been being proved to be useful information for diagnosis of lung disease such as asthma and chronic obstructive pulmonary disease (COPD). For example, J. Zhang et al. "Correlation between levels of exhaled hydrogen sulfide and airway inflammatory phenotype in patients with chronic persistent asthma", Respirology. 28 August 2014; 19: 1165-1169 discusses correlation between airway chronic inflammation and hydrogen sulfide in exhaled breath of patients with chronic persistent asthma. J. Zhang et al. "Exhaled Hydrogen Sulfide Predicts Airway Inflammation Phenotype in COPD", Respiratory Care. 29 January 2015; 60(2): 251-258 discusses a role of hydrogen sulfide in exhaled breath as a marker of airway inflammation and correlation with COPD severity. Furthermore, S. Yun et al. "Exhaled hydrogen sulfide in patients with chronic obstructive pulmonary disease and its correlation with exhaled nitric oxide", Chinese Medical Journal 2013; 126 (17): 3240-3244 discusses correlation between hydrogen sulfide in exhaled breath and COPD.

Under this background, there have been requirements for technologies for accurately measuring the concentration of sulfide gas contained in exhaled breath. According to an inventors' study, consideration should be given to the following issues in concentration measurement of sulfide gas contained in exhaled breath.

First, it is desired to suppress mixing of sulfide gas generated in tissues other than the lungs into exhaled breath in the concentration measurement of the sulfide gas contained in the exhaled breath. For appropriate diagnosis of lung disease, it is important to obtain information concerning generation of sulfide gas in the lungs. Meanwhile, according to an inventors' knowledge, sulfide gas may be generated in organs other than the lungs, such as the nasal cavities and the stomach. Accordingly, it is useful for lung disease diagnosis to suppress mixing of sulfide gas generated in tissues other than the lungs into exhaled breath.

It is also desired to measure the concentration of the sulfide gas contained in the exhaled breath while variations in the sulfide gas concentration resulting from causes other than the pathology of lung disease are suppressed. According to an inventors' knowledge, the concentration of sulfide gas contained in exhaled breath depends on the flow rate of the exhaled breath as well as the pathology of the lung disease. Accordingly, it is desired to measure the concentration of the sulfide gas contained in the exhaled breath while variations in the flow rate of the exhaled breath of the subject are suppressed.

It is further desired to suppress loss of the sulfide gas contained in the collected exhaled breath in the concentration measurement of the sulfide gas. The concentration of sulfide gas contained in exhaled breath is not so high, whereas the concentration of the sulfide gas contained in the exhaled breath decreases over time due to absorption and decomposition. This means information useful for lung disease diagnosis vanishes.

It is desired to satisfy at least one of these three needs in concentration measurement of sulfide gas in exhaled breath.

It should be noted that devices for concentration detection of hydrogen sulfide in exhaled breath are disclosed in JP 2014-522973 A, JP 2015-526732 A, JP 2015-526733 A and WO 2017/040546 A1.

WO 2017/040546 A1 discloses a portable SIBO testing system for convenient sampling of intestinal gases exhaled from a patient's breath, which testing system allows the patient to frequently test its breath gas levels while simultaneously enter and store information about the time and content of meals consumed prior to testing. The testing system comprises a breath collector, a sorbent based detection module in gaseous communication with the breath collector that outputs data indicative of the concentration of gases such as H₂S entering the detection module, a control system for determining a concentration of gases detected by the sorbent based detection module, a display that outputs the determined concentration of gases, and optionally a flow meter, a flow regulator, a moisture regulator or/and a carbon dioxide sensor.

US 2014/0305810 A1 discloses a device for analyzing the concentration of NO in exhaled breath of a subject. In one embodiment, the device comprises, among others, a pressure sensor, an NO scrubber, an exhalation flow controller, a buffer chamber, a sample pump or fan, and an NO sensor, wherein the sample pump or fan is connected to the inlet of the NO sensor and the NO sensor is an electrochemical sensor.

US 2017/0065208 A1 discloses an apparatus for determining the concentration of NOₓ from a gas stream such as exhaled breath. In one embodiment, the apparatus comprises a disposable mouthpiece and a detection box, wherein the detection box includes, among others, a desiccant, a flow sensor, a humidity sensor, a pressure sensor, a pump and a microchannel rector/sensor assembly, the pump being connected to the inlet of the microchannel rector/sensor assembly.

### Citation List

### Patent Literature

[Patent Literature 1] JP2014-522973 A
[Patent Literature 2] JP 2015-526732 A
[Patent Literature 3] JP 2015-526733 A
[Patent Literature 4] WO 2017/040546 A1
[Patent Literature 5] US 2014/0305810 A1
[Patent Literature 6] US 2017/0065208 A1

### Non-Patent Literature

[Non-Patent Literature 1] J. Zhang et al. Correlation between levels of exhaled hydrogen sulfide and airway inflammatory phenotype in patients with chronic persistent asthma. Respirology. 28 August 2014; 19: 1165-1169.
[Non-Patent Literature 2] J. Zhang et al. Exhaled Hydrogen Sulfide Predicts Airway Inflammation Phenotype in COPD. Respiratory Care. 29 January 2015; 60(2): 251-258.
[Non-Patent Literature 3] S. Yun et al. Exhaled hydrogen sulfide in patients with chronic obstructive pulmonary disease and its correlation with exhaled nitric oxide. Chinese Medical Journal 2013; 126 (17) : 3240-3244.

### Summary

Accordingly, an objective of the present invention is to achieve, in concentration measurement of sulfide gas in exhaled breath, at least one of: suppression of mixing of sulfide gas generated in tissues other than the lungs into exhaled breath; suppression of variations in the concentration of sulfide gas resulting from causes other than the pathology of lung disease; and suppression of loss of sulfide gas from collected exhaled breath. Other objectives and new features of the present invention would be understood by a skilled person from the following disclosure. The invention is defined by the claims.

In a first aspect, the invention thus relates to a sulfide gas concentration measuring device as defined in claim 1. The sulfide gas concentration measuring device comprises: an exhaled breath collection tool to be put to a subject to introduce exhaled breath of the subject thereinto; a pressure regulator comprising an inlet port connected to the exhaled breath collection tool; a sulfide gas sensor connected to an outlet port of the pressure regulator to measure a concentration of sulfide gas in the exhaled breath discharged from the outlet port; and a pressure measuring device configured to measure a pressure in the inlet port of the pressure regulator to make the subject aware of the measured pressure, wherein the pressure in the outlet port of the pressure regulator is equal to the pressure in an inlet port of the sulfide gas sensor, and wherein the pressure regulator, by keeping the pressure in the outlet port of the pressure regulator at a predetermined set pressure, keeps the pressure in the inlet port of the sulfide gas sensor at the predetermined set pressure.

In a second aspect, the invention relates to a sulfide gas concentration measuring method as defined in claim 5. The sulfide gas concentration measuring method uses the sulfide gas concentration measuring device of the first aspect and comprises the steps of: putting the exhaled breath collection tool to a subject; introducing exhaled breath of the subject into the inlet port of the pressure regulator via the exhaled breath collection tool while a pressure in the inlet port of the pressure regulator is measured to make the subject aware of the measured pressure; introducing the exhaled breath discharged from the outlet port of the pressure regulator into the sulfide gas sensor; and measuring the concentration of sulfide gas contained in the exhaled breath of the subject by the sulfide gas sensor.

The present invention effectively achieves, in concentration measurement of sulfide gas in exhaled breath, at least one of: suppression of mixing of sulfide gas generated in tissues other than the lungs into exhaled breath; suppression of mixing of sulfide gas generated in tissues other than the lungs into exhaled breath; and suppression of loss of sulfide gas from collected exhaled breath.

### Brief Description of Drawings

FIG. 1 is a block diagram showing the configuration of a sulfide gas concentration measuring device, according to one embodiment;
FIG. 2 is a diagram schematically showing a use method of the sulfide gas concentration measuring device, according to this embodiment;
FIG. 3 is a block diagram showing an operation of the sulfide gas concentration measuring device in concentration measurement of sulfide gas contained in exhaled breath, according to this embodiment;
FIG. 4 is a block diagram showing an operation of the sulfide gas concentration measuring device in calibration of a sulfide gas sensor, according to this embodiment;
FIG. 5 is a block diagram showing an operation of the sulfide gas concentration measuring device in cleaning of the sulfide gas sensor, according to this embodiment; and
FIG. 6 is a block diagram showing the configuration of a sulfide gas concentration measuring device configured to perform automatic cleaning of the sulfide gas sensor after measuring the concentration of sulfide gas in exhaled breath, according to this embodiment.

### Description of Embodiments

In the following, a description is given of embodiments of a sulfide gas concentration measuring device and a sulfide gas concentration measuring method according to the present invention, with reference to the attached drawings.

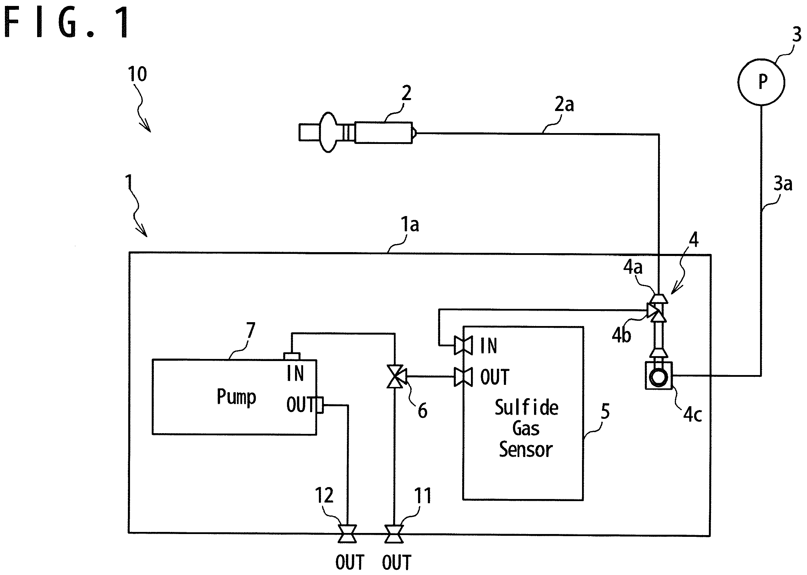

FIG. 1 is a block diagram showing the configuration of a sulfide gas concentration measuring device 10, according to one embodiment. The sulfide gas concentration measuring device 10 comprises a main unit 1, a mouth piece 2, and a pressure gauge 3. As described later in detail, the sulfide gas concentration measuring device 10 is configured to measure the concentration of sulfide gas (for example, hydrogen sulfide) in exhaled breath of a subject. The mouth piece 2 is used as an exhaled breath collection tool to be put to the subject for introducing the exhaled breath of the subject into the sulfide gas concentration measuring device 10. A mask may be used in place of the mouth piece 2.

The main unit 1 comprises a housing 1a which accommodates a pressure regulator 4, a sulfide gas sensor 5, a three-way valve 6, and a pump 7.

The pressure regulator 4 comprises an inlet port 4a and an outlet port 4b. The inlet port 4a is connected to the mouth piece 2 via an exhaled breath line 2a. The subject's exhaled breath introduced into the mouth piece 2 is supplied to the inlet port 4a of the pressure regulator 4. The pressure regulator 4 is configured to discharge the subject's exhaled breath received by the inlet port 4a from the outlet port 4b with a predetermined set pressure. The pressure regulator 4 is configured to adjust the pressure in the outlet port 4b (the secondary pressure) to the set pressure, when the pressure in the inlet port 4a (the primary pressure) is sufficiently high. It should be noted that such operation is common as the operation of a pressure regulator. The outlet port 4b of the pressure regulator 4 is connected to the sulfide gas sensor 5.

In this embodiment, the pressure regulator 4 further comprises a pressure measurement port 4c. The pressure measurement port 4c is communicated with the inlet port 4a, and accordingly the pressure in the pressure measurement port 4c is equal to that in the inlet port 4a. In this embodiment, the pressure measurement port 4c is connected to the pressure gauge 3 via a pressure measurement line 3a. In this embodiment, the pressure gauge 3, which is connected to the pressure measurement port 4c, is used as a pressure measuring device which measures the pressure in the inlet port 4a of the pressure regulator 4.

The sulfide gas sensor 5 receives the exhaled breath of the subject from the outlet port 4b of the pressure regulator 4 and measures the concentration of sulfide gas contained in the exhaled breath of the subject. In one embodiment, a controlled potential electrolysis sensor may be used as the sulfide gas sensor 5. In an alternative embodiment, to make it possible to determine the type of sulfide gas (for example, to distinguish hydrogen sulfide from other sulfide gas such as sulfur dioxide), an optical gas sensor configured to perform gas detection based on optical absorption may be used.

The three-way valve 6 is configured as a switch valve comprising one inlet port and two outlet ports. The inlet port of the three-way valve 6 is connected to the outlet port of the sulfide gas sensor 5. One of the outlet ports of the three-way valve 6 is connected to an external outlet port 11, and the other outlet port is connected to the pump 7. The external outlet port 11 is open to the atmosphere. The three-way valve 6 connects the outlet port of the sulfide gas sensor 5 to the pump 7 or the external outlet port 11 depending on the manipulation.

The pump 7 comprises an inlet port connected to the outlet port of the three-way valve 6 and an outlet port connected to an external outlet port 12. The external outlet port 12 is open to the atmosphere. As described later, the pump 7 is used for calibration of the sulfide gas sensor 5.

The connection between the pressure gauge 3 and the inlet port 4a may be variously modified, although the pressure gauge 3 is connected to the pressure measurement port 4c, which is communicated with the inlet port 4a, and the pressure in the inlet port 4a of the pressure regulator 4 is measured by the pressure gauge 3 in this embodiment. For example, the pressure gauge 3 may be connected to the exhaled breath line 2a.

A description is then given of the operation of the sulfide gas concentration measuring device 10, according to this embodiment.

The sulfide gas concentration measuring device 10 in this embodiment is used to measure the concentration of sulfide gas (for example, hydrogen sulfide) contained in exhaled breath. As described above, the concentration of sulfide gas contained in the exhaled breath is useful information for diagnosis of lung disease such as asthma and chronic obstructive pulmonary disease (COPD). The sulfide gas concentration measuring device 10 in this embodiment measures the concentration of the sulfide gas contained in the exhaled breath of the subject through a method suitable for lung disease diagnosis, as described in the following.

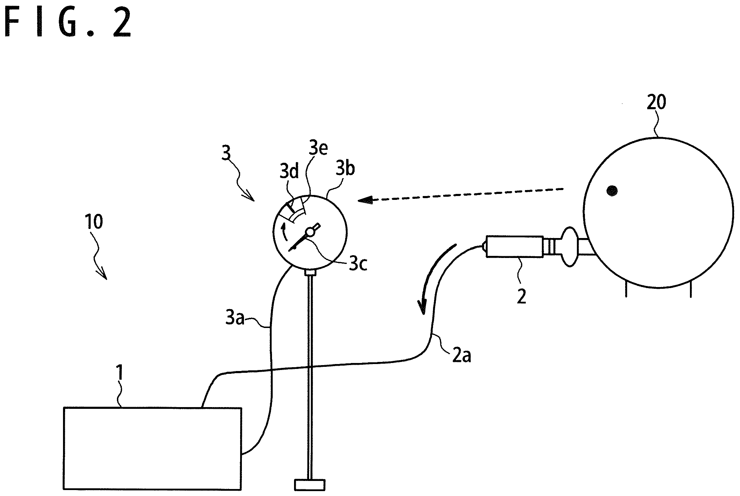

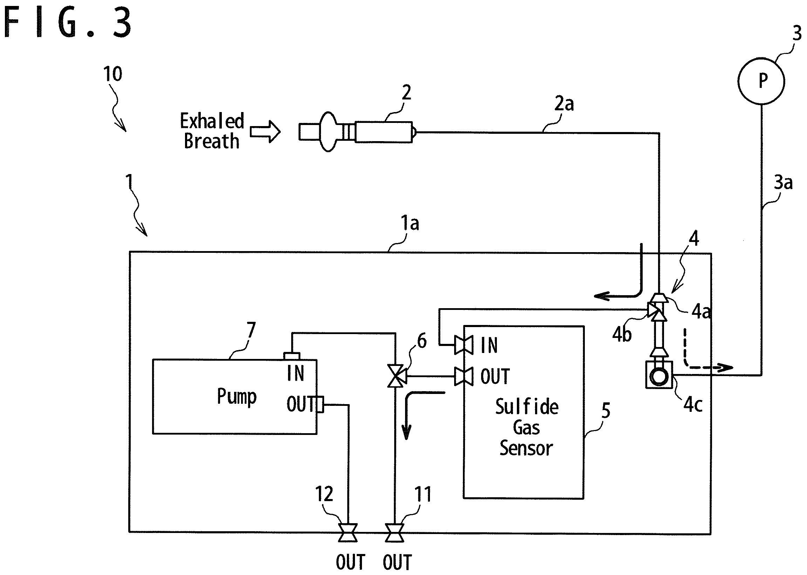

FIG. 2 is a diagram schematically showing a use method of the sulfide gas concentration measuring device 10 when the concentration of sulfide gas contained in exhaled breath of a subject is measured, and FIG. 3 is a block diagram showing the operation of the sulfide gas concentration measuring device 10 when the concentration of the sulfide gas is measured. When the concentration of the sulfide gas contained in the exhaled breath of the subject is measured, the outlet port of the sulfide gas sensor 5 is connected to the external outlet port 11 by the three-way valve 6. It is not necessary to operate the pump 7.

As shown in FIG. 2, when the concentration of sulfide gas contained in exhaled breath of a subject 20 is measured, the subject 20 has the mouth piece 2 put thereto, and the subject 20 is asked to blow exhaled breath into the mouth piece 2.

While the subject 20 blows the exhaled breath into the mouth piece 2, the subject 20 is made aware of the pressure measured by the pressure gauge 3 and asked to blow the exhaled breath so that the pressure indicated by the pressure gauge 3 is adjusted to a specific target pressure (for example, 1.5 kPa) or to a specific target pressure range (for example, a specific pressure range with a center value of 1.5 kPa.) For example, the display unit 3b of the pressure gauge 3 may be shown to the subject 20 and the subject 20 may be asked to blow the exhaled breath into the mouth piece 2 so that the pressure indicated by the pressure gauge 3 is adjusted to the target pressure or the target pressure range. Since the pressure gauge 3 measures the pressure in the inlet port 4a of the pressure regulator 4, this operation resultingly adjusts the pressure in the inlet port 4a to at least the vicinity of the target pressure or the target pressure range. The target pressure or the target pressure range is determined as being sufficient for the pressure regulator 4 to adjust the pressure in the outlet port 4b to a predetermined set pressure.

The subject's exhaled breath blown into the mouth piece 2 is introduced into the inlet port 4a of the pressure regulator 4, discharged from the outlet port 4b with its pressure reduced to the set pressure, and introduced to the inlet port of the sulfide gas sensor 5 from the outlet port 4b of the pressure regulator 4 with the set pressure set to the pressure regulator 4. When the pressure in the inlet port 4a is sufficiently high, this allows the pressure regulator 4 to keep the pressure in the outlet port 4b at the predetermined set pressure. The sulfide gas sensor 5 receives the exhaled breath of the subject 20 from the outlet port 4b of the pressure regulator 4 and measures the concentration of the sulfide gas contained in the exhaled breath. The exhaled breath which has been subjected to the concentration measurement of sulfide gas is discharged from the outlet port of the sulfide gas sensor 5 and then discharged to the external atmosphere via the three-way valve 6 and the external outlet port 11.

The operation which involves asking the subject 20 to blow the exhaled breath into the mouth piece 2 while the subject 20 is made aware of the pressure measured by the pressure gauge 3 is effective for suppressing mixing of sulfide gas generated in tissues other than the lungs into the exhaled breath. The route between the lung airways and the mouth is open to the nasal cavities and the stomach in a usual state. In this state, sulfide gas generated in the nasal cavities and the stomach may be mixed into the exhaled breath; however, by asking the subject 20 to keep the pressure measured by the pressure gauge 3 at the target pressure or in the target pressure range, it is possible to introduce the exhaled breath into the mouth piece 2 while the openings to the nasal cavities and the stomach are closed. To keep the pressure measured by the pressure gauge 3 at a certain high pressure, the subject 20 is necessary to exert a force of a certain magnitude to exhale the breath. When the subject 20 exerts a force of a certain magnitude to exhale the breath, the openings to the nasal cavities and the stomach existing along the route between the lung airways and the mouth are closed in the body of the subject 20. This makes it possible to suppress mixing of sulfide gas generated in tissues other than the lungs into the exhaled breath. The configuration of the sulfide gas concentration measuring device 10 according to this embodiment, in which the pressure of the inlet port 4a of the pressure regulator 4 is measured by the pressure gauge 3, preferably suppresses mixing of sulfide gas generated in tissues other than the lungs into the exhaled breath through the above-described operation.

The operation which involves asking the subject 20 to blow the exhaled breath into the mouth piece 2 while the subject 20 is made aware of the pressure measured by the pressure gauge 3 is also effective for suppressing variations in the flow rate of the exhaled breath of the subject 20 and obtaining information useful for lung disease diagnosis. As described above, the concentration of the sulfide gas contained in the exhaled breath depends on the flow rate of the exhaled breath from the subject 20. It is possible to suppress an influence of variations in the flow rate of the exhaled breath by measuring the concentration of the sulfide gas while asking subject 20 to exhale the breath so that the pressure indicated by the pressure gauge 3 is adjusted to the specific pressure. The configuration of the sulfide gas concentration measuring device 10 according to this embodiment, in which the pressure of the inlet port 4a of the pressure regulator 4 is measured by the pressure gauge 3, is suitable for suppressing an influence of variations in the flow rate of the exhaled breath.

Furthermore, the sulfide gas concentration measuring device 10 according to this embodiment achieves in-situ measurement of the concentration of the sulfide gas contained in the exhaled breath of the subject 20 with accuracy sufficient for lung disease diagnosis. To improve the accuracy of the sulfide gas concentration detection with the sulfide gas sensor 5, it is desired that the gas to be subjected to the detection flows into the sulfide gas sensor 5 at a constant flow rate. In this embodiment, the pressure in the outlet port 4b, that is, the pressure in the inlet port of the sulfide gas sensor 5 is kept substantially constant by the operation of the pressure regulator 4, and accordingly the exhaled breath of the subject flows into the sulfide gas sensor 5 at a substantially constant flow rate. This allows the configuration according to this embodiment to achieve in-situ measurement of the sulfide gas contained in the exhaled breath of the subject 20 with accuracy sufficient for lung disease diagnosis.

The in-situ measurement ability of the concentration of the sulfide gas contained in the exhaled breath of the subject 20 is effective for suppressing loss of the sulfide gas from the collected exhaled breath. As described above, the concentration of sulfide gas in exhaled breath decreases over time due to absorption and decomposition. For example, a system configured to blow exhaled breath into a bag and measure the concentration of sulfide gas contained in the exhaled breath accumulated in the bag experiences a decrease in the concentration of the sulfide gas due to absorption of the sulfide gas into the bag. This undesirably implies that information used for lung disease diagnosis is lost. The sulfide gas concentration measuring device 10 according to this embodiment achieves in-situ measurement of the sulfide gas concentration, suppressing loss of the sulfide gas from the collected exhaled breath.

Various methods may be used to make the subject 20 aware of the pressure measured by the pressure gauge 3. The subject 20 may be made visually aware of the pressure measured by the pressure gauge 3. The subject 20 may be made aurally aware of the pressure (for example, by generating sound corresponding to the pressure). In one embodiment, the display unit 3b of the pressure gauge 3 may be shown to the subject 20. In this case, the display unit 3b may comprise a display element 3c (e.g., an indicator) visually indicating the measured pressure, a marking 3d indicating the target pressure and/or a marking 3e indicating the target pressure range, where the display element 3c and the markings 3d and 3e are all configured to be visually perceivable. In this case, the subject 20 may be asked to blow the exhaled breath into the mouth piece 2 so that the position of the display element 3c is adjusted to match the position of the marking 3d indicating the target pressure or the marking 3e indicating the target pressure range.

The pump 7 is used for calibration and cleaning of the sulfide gas sensor 5.

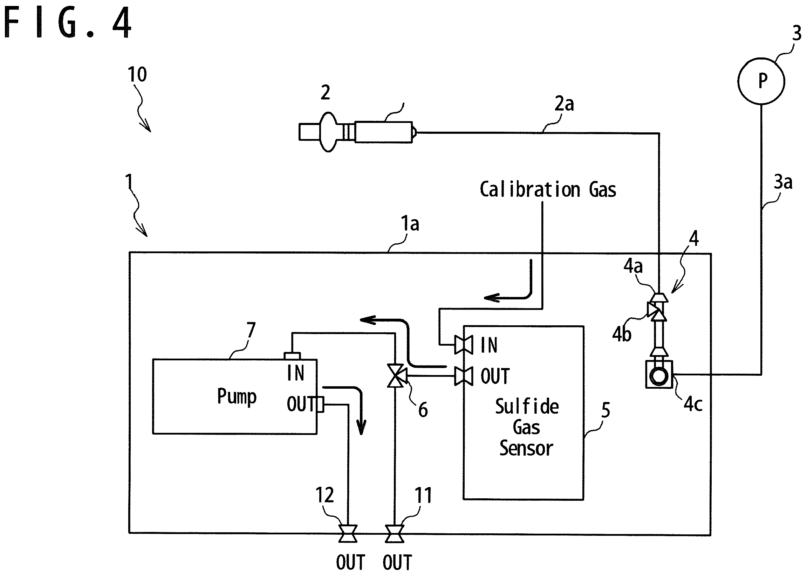

FIG. 4 is a block diagram showing the operation of the sulfide gas concentration measuring device 10 according to this embodiment in performing calibration of the sulfide gas sensor 5. When the sulfide gas sensor 5 is calibrated, the three-way valve 6 is set to connect the outlet port of the sulfide gas sensor 5 to the pump 7. Additionally, the inlet port of the sulfide gas sensor 5 is disconnected from the outlet port 4b of the pressure regulator 4 and connected to a gas source (not shown) which supplies calibration gas.

In the calibration, the pump 7 is operated to suck gas therein at a constant flow rate and calibration gas containing sulfide gas with a known concentration is introduced into the inlet port of the sulfide gas sensor 5. Since the pump 7 connected to the outlet port of the sulfide gas sensor 5 is operated to suck gas therein at the constant flow rate, the sulfide gas is resultingly introduced into the sulfide gas sensor 5 at a constant flow rate. The sulfide gas sensor 5 measures the concentration of the sulfide gas contained in the calibration gas in this state. The sulfide gas sensor 5 is calibrated by using the measured sulfide gas concentration of the calibration gas.

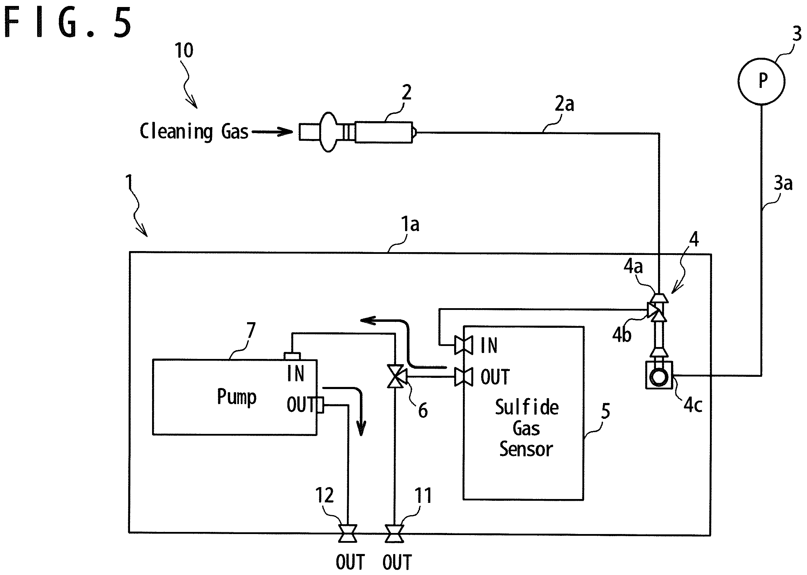

FIG. 5 is a block diagram showing the operation of the sulfide gas concentration measuring device 10 according to this embodiment in performing cleaning of the sulfide gas sensor 5. The three-way valve 6 is set to connect the outlet port of the sulfide gas sensor 5 to the pump 7, also when the cleaning of the sulfide gas sensor 5 is performed.

In the cleaning, cleaning gas is introduced into the mouth piece 2 in a state in which the pump 7 is operated. This achieves introducing the cleaning gas into the inlet port of the sulfide gas sensor 5 via the exhaled breath line 2a and the pressure regulator 4. Since the pump 7 is operated, the cleaning gas which has cleaned the internal of the sulfide gas sensor 5 is sucked by the pump 7 from the outlet port of the sulfide gas sensor 5 and discharged from the external outlet port 12. This operation achieves cleaning of the route from the mouth piece 2 to the inlet port of the sulfide gas sensor 5 and the internal of the sulfide gas sensor 5.

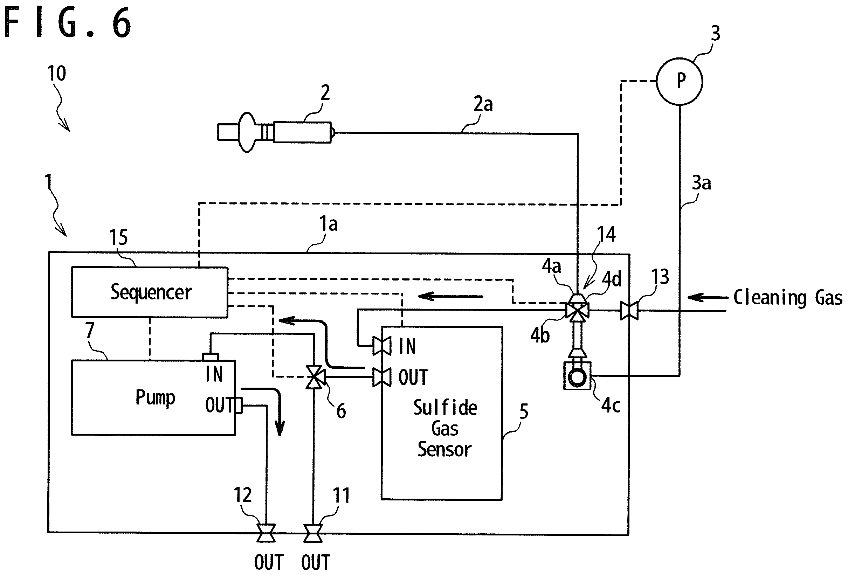

The sulfide gas concentration measuring device 10 may be configured to automatically perform cleaning of the sulfide gas sensor 5 after the sulfide gas concentration measurement of the exhaled breath. FIG. 6 is a block diagram showing the configuration of a sulfide gas concentration measuring device 10 thus configured. In the configuration shown in FIG. 6, a pressure regulator 14 is provided in place of the pressure regulator 4 shown in FIG. 1, and the main unit 1 additionally comprises an external connection port 13 and a sequencer 15. The sequencer 15 is used as a control device which monitors the pressure measured by the pressure gauge 3 and controls the sulfide gas sensor 5, the three-way valve 6, the pump 7, and the pressure regulator 14.

The pressure regulator 14 comprises a gas introduction port 4d in addition to an inlet port 4a, an outlet port 4b, and a pressure measurement port 4c. The gas introduction port 4d is connected to the external connection port 13. The external connection port 13 is connected to a cleaning gas source (not shown) which supplies cleaning gas.

The sulfide gas concentration measuring device 10 configured as shown in FIG. 6 operates as follows. The sequencer 15 outputs an optical or acoustic output to prompt the subject to blow exhaled breath into the mouth piece 2 when starting measurement of the exhaled breath. At this moment, the sequencer 15 sets the three-way valve 6 to connect the outlet port of the sulfide gas sensor 5 to the external outlet port 11 and closes the gas introduction port 4d of the pressure regulator 14. Meanwhile, the sequencer 15 stops the operation of the pump 7. When detecting from the pressure measured by the pressure gauge 3 that the subject blows exhaled breath into the mouth piece 2, the sequence 15 controls the sulfide gas sensor 5 to measure the concentration of the sulfide gas contained in the exhaled breath.

When completing the concentration measurement of the sulfide gas contained in the exhaled breath, the sequencer 15 operates to clean the sulfide gas sensor 5. The sequencer 15 sets the pressure regulator 14 so that the gas introduction port 4d communicates with the outlet port 4b. This achieves communicating the external connection port 13, which is connected to the cleaning gas source, with the inlet port of the sulfide gas sensor 5. Furthermore, the sequencer 15 sets the three-way valve 6 to connect the outlet port of the sulfide gas sensor 5 to the pump 7 and operates the pump 7. This allows the cleaning gas to be introduced into the sulfide gas sensor 5 from the external connection port 13 via the pressure regulator 14. This achieves cleaning of the internal of the sulfide gas sensor 5 and the line connected to the inlet port of the sulfide gas sensor 5.

A three-way valve (switch valve) controlled by the sequencer 15 may be disposed on the line connecting the pressure regulator 14 to the sulfide gas sensor 5, in place of providing the gas introduction port 4d for the pressure regulator 14. In this case, the three-way valve connects the inlet port of the sulfide gas sensor 5 to one of the outlet port 4b of the pressure regulator 14 and the external connection port 13 under the control of the sequencer 15. When the sulfide gas concentration of the exhaled breath is measured, this three-way connects the inlet port of the sulfide gas sensor 5 to the outlet port 4b of the pressure regulator 14. When the sulfide gas sensor 5 is cleaned, the three-way connects the inlet port of the sulfide gas sensor 5 to the external connection port 13, which is connected to the cleaning gas source.

Although embodiments of the sulfide gas concentration measuring device according to the present invention have been specifically disclosed, the present invention must not be construed as being limited to the above-described embodiments. It would be apparent to a skilled person that the present invention can be implemented with various modifications within the scope of the claims.

### Reference Signs List

1: main unit
1a: housing
2: mouth piece
2a: exhaled breath line
3: pressure gauge
3a: pressure measurement line
3b: display unit
3c: display element
3d, 3e: marking
4: pressure regulator
4a: inlet port
4b: outlet port
4c: pressure measurement port
4d: gas introduction port
5: sulfide gas sensor
6: three way valve
7: pump
10: sulfide gas measuring device
11, 12: external outlet port
13: external connection port
14: pressure regulator
15: sequencer
20: subject

## Claims

1. A sulfide gas concentration measuring device (10), comprising:
an exhaled breath collection tool (2) to be put to a subject (20) to introduce exhaled breath of the subject (20) thereinto;
a pressure regulator (4) comprising an inlet port (4a) receiving the exhaled breath from the exhaled breath collection tool (2);
a sulfide gas sensor (5) connected to an outlet port (4b) of the pressure regulator (4) to measure a concentration of sulfide gas in the exhaled breath discharged from the outlet port (4b); and
**characterized by** a pressure measuring device (3) configured to measure a pressure in the inlet port (4a) of the pressure regulator (4) to make the subject (20) aware of the measured pressure, and further **characterized in that** the pressure in the outlet port (4b) of the pressure regulator (4) is equal to the pressure in an inlet port of the sulfide gas sensor (5), and
the pressure regulator (4), by keeping the pressure in the outlet port (4b) of the pressure regulator (4) at a predetermined set pressure, keeps the pressure in the inlet port of the sulfide gas sensor (5) at the predetermined set pressure.

2. The sulfide gas concentration measuring device (10) according to claim 1, wherein the pressure measuring device (3) comprises a display unit (3b), and
wherein the display unit (3b) comprises:
a display element (3c) visually indicating the measured pressure; and
a marking (3d, 3e) visually indicating a target pressure or a target pressure range.

3. The sulfide gas concentration measuring device (10) according to claim 1 or 2, further comprising:
an external outlet port (11, 12) open to an atmosphere;
a pump (7); and
a switch valve (6) configured to connect an outlet port of the sulfide gas sensor (5) to a selected one of the external outlet port (11, 12) and the pump (7), the exhaled breath being discharged from the outlet port of the sulfide gas sensor (5).

4. The sulfide gas concentration measuring device (10) according to claim 3, further comprising:
an external connection port (13) to be connected to a cleaning gas source supplying cleaning gas and configured to be connectable to the inlet port of the sulfide gas sensor (5), the exhaled breath being introduced into the inlet port of the sulfide gas sensor (5); and
a control device (15) which controls the sulfide gas sensor (5), the pump (7), and the switch valve (6) and controls a connection between the external connection port (13) and the inlet port of the sulfide gas sensor (5),
wherein, when the concentration of the sulfide gas in the exhaled breath is measured, the control device (15) disconnects the external connection port (13) from the inlet port of the sulfide gas sensor (5), sets the switch valve (6) to connect the outlet port of the sulfide gas sensor (5) to the external outlet port (13), and causes the sulfide gas sensor (5) to measure the concentration of the sulfide gas in the exhaled breath in a state in which the pump (7) is stopped, and
wherein, after the measurement of the concentration of the sulfide gas in the exhaled breath is completed, the control device (15) connects the external connection port (13) to the inlet port of the sulfide gas sensor (5) and operates the pump (7) in a state in which the switch valve (6) is set to connect the outlet port of the sulfide gas sensor (5) to the pump (7).

5. A sulfide gas concentration measuring method using the sulfide gas concentration measuring device (10) according to any one of claims 1 to 4, the method comprising the steps of:
putting the exhaled breath collection tool (2) to a subject (20);
introducing exhaled breath of the subject (20) into the inlet port (4a) of the pressure regulator (4) via the exhaled breath collection tool (2) while a pressure in the inlet port (4a) of the pressure regulator (4) is measured and the subject (20) is made aware of the measured pressure;
introducing the exhaled breath discharged from the outlet port (4b) of the pressure regulator (4) into the sulfide gas sensor (5); and
measuring a concentration of sulfide gas contained in the exhaled breath of the subject (20) by the sulfide gas sensor (5).

6. The sulfide gas concentration measuring method according to claim 5, wherein the pressure in the inlet port (4a) is measured by a pressure measuring device (3) comprising a display unit (3b), and
wherein the display unit (3b) comprises:
a display element (3c) visually indicating the measured pressure; and
a marking (3d, 3e) visually indicating a target pressure or a target pressure range.

## Patentansprüche

1. Ein Sulfidgaskonzentrationsmessgerät (10), umfassend:
ein Ausatemluft-Sammelgerät (2), das an eine Person (20) angelegt werden kann, um die ausgeatmeten Atemluft der Person (20) einzuleiten;
einen Druckregler (4), der einen Einlassanschluss (4a) umfasst, der die ausgeatmete Atemluft vom Ausatemluft-Sammelgerät (2) aufnimmt;
einen Sulfidgassensor (5), der mit einer Auslassöffnung (4b) des Druckreglers (4) verbunden ist, um eine Konzentration von Sulfidgas in der ausgeatmeten Atemluft zu messen, die von der Auslassöffnung (4b) abgegeben wird; und
**gekennzeichnet durch** ein Druckmessgerät (3), das konfiguriert ist, um einen Druck im Einlassanschluss (4a) des Druckreglers (4) zu messen, um die Person (20) auf den gemessenen Druck aufmerksam zu machen,
und weiter **gekennzeichnet dadurch, dass** der Druck in der Auslassöffnung (4b) des Druckreglers (4) gleich dem Druck in einer Einlassöffnung des Sulfidgassensors (5) ist, und
dadurch, dass der Druckregler (4), in dem er den Druck im Auslassanschluss (4b) des Druckreglers (4) auf einem vorgegebenen Einstelldruck hält, den Druck im Einlassanschluss des Sulfidgassensors (5) auf dem vorgegebenen Einstelldruck hält.

2. Das Sulfidgaskonzentrationsmessgerät (10) nach Anspruch 1, wobei das Druckmessgerät (3) eine Anzeigeeinheit (3b) umfasst
wobei die Anzeigeeinheit (3b) umfasst:
ein Anzeigeelement (3c), das den gemessenen Druck visuell anzeigt; und
eine Markierung (3d, 3e), die einen Zieldruck oder einen Zieldruckbereich optisch anzeigt.

3. Das Sulfidgaskonzentrationsmessgerät (10) nach Anspruch 1 oder 2, weiterhin umfassend:
eine externe Auslassöffnung (11, 12), die zur Atmosphäre hin offen ist;
eine Pumpe (7); und
ein Schaltventil (6), das konfiguriert ist, um einen Auslassanschluss des Sulfidgassensors (5) mit dem externen Auslassanschluss (11, 12) oder der Pumpe (7) zu verbinden, wobei der ausgeatmeten Atemluft über den Auslassanschluss des Sulfidgassensors (5) abgegeben wird.

4. Das Sulfidgaskonzentrationsmessgerät (10) nach Anspruch 3, weiterhin umfassend:
einen externen Verbindungsanschluss (13), der an eine Reinigungsgasquelle angeschlossen werden kann, die Reinigungsgas liefert, und konfiguriert ist, um mit dem Einlassanschluss des Sulfidgassensors (5) verbunden zu werden, wobei die ausgeatmete Atemluft in den Einlassanschluss des Sulfidgassensors (5) eingeleitet wird; und
eine Steuervorrichtung (15), die den Sulfidgassensor (5), die Pumpe (7) und das Schaltventil (6) steuert, und eine Verbindung zwischen dem externen Verbindungsanschluss (13) und dem Einlassanschluss des Sulfidgassensors (5) steuert,
wobei, wenn die Konzentration des Sulfidgases in der ausgeatmeten Atemluft gemessen wird, die Steuervorrichtung (15) den externen Verbindungsanschluss (13) vom Einlassanschluss des Sulfidgassensors (5) trennt, und das Schaltventil (6) einstellt. um den Auslassanschluss des Sulfidgassensors (5) mit dem externen Auslassanschluss (13) zu verbinden, und den Sulfidgassensor (5) veranlasst, die Konzentration des Sulfidgases in der ausgeatmeten Atemluft in einem Zustand zu messen, in dem die Pumpe (7) angehalten ist, und
wobei, nachdem die Messung der Konzentration des Sulfidgases in der ausgeatmeten Atemluft abgeschlossen ist, die Steuervorrichtung (15) den externen Verbindungsanschluss (13) mit dem Einlassanschluss des Sulfidgassensors (5) verbindet, und die Pumpe in einem Zustand betreibt (7), in dem das Schaltventil (6) so eingestellt ist, dass es den Auslassanschluss des Sulfidgassensors (5) mit der Pumpe (7) verbindet.

5. Verfahren zur Messung der Sulfidgaskonzentration unter Verwendung des Sulfidgaskonzentrationsmessgeräts (10) nach einem der Ansprüche 1 bis 4, wobei das Verfahren die folgenden Schritte umfasst:
Anbringen des Ausatemluft-Sammelgeräts (2) an einem Subjekt (20);
Einleiten der ausgeatmeten Atemluft der Person (20) in die Einlassöffnung (4a) des Druckreglers (4) über das Ausatemluft-Sammelgerät (2), während ein in der Einlassöffnung (4a) des Druckreglers (4) herrschender Druck gemessen und der Proband (20) auf den gemessenen Druck aufmerksam gemacht wird;
Einleiten der ausgeatmeten Atemluft, die von der Auslassöffnung (4b) des Druckreglers (4) abgegeben wird, in den Sulfidgassensor (5); und
Messen einer Konzentration von Sulfidgas, das in der ausgeatmeten Atemluft der Person (20) enthalten ist, durch den Sulfidgassensor (5).

6. Verfahren zur Messung der Sulfidgaskonzentration nach Anspruch 5, wobei der Druck in der Einlassöffnung (4a) durch eine Druckmessvorrichtung (3) gemessen wird, die eine Anzeigeeinheit (3b) umfasst
wobei die Anzeigeeinheit (3b) umfasst:
ein Anzeigeelement (3c), das den gemessenen Druck visuell anzeigt; Und
eine Markierung (3d, 3e), die einen Zieldruck oder einen Zieldruckbereich optisch anzeigt.

## Revendications

1. Dispositif de mesure de la concentration en gaz de sulfure (10), comprenant :
un outil de collecte d'air expiré (2) à placer sur un sujet (20) pour introduire de l'air expiré du sujet (20) dans celui-ci ;
un régulateur de pression (4) comprenant un orifice d'entrée (4a) recevant l'air expiré de l'outil de collecte d'air expiré (2) ;
un capteur de gaz de sulfure (5) connecté à un orifice de sortie (4b) du régulateur de pression (4) pour mesurer une concentration en gaz de sulfure dans l'air expiré évacué de l'orifice de sortie (4b) ; et
**caractérisé par**
un dispositif de mesure de pression (3) configuré pour mesurer une pression dans l'orifice d'entrée (4a) du régulateur de pression (4) pour avertir le sujet (20) de la pression mesurée, et en outre **caractérisé en ce que** la pression dans l'orifice de sortie (4b) du régulateur de pression (4) est égale à la pression dans un orifice d'entrée du capteur de gaz de sulfure (5), et
le régulateur de pression (4), en maintenant la pression dans l'orifice de sortie (4b) du régulateur de pression (4) à une pression de consigne prédéterminée, maintient la pression dans l'orifice d'entrée du capteur de gaz de sulfure (5) à la pression de consigne prédéterminée.

2. Dispositif de mesure de la concentration en gaz de sulfure (10) selon la revendication 1, dans lequel le dispositif de mesure de pression (3) comprend une unité d'affichage (3b), et
dans lequel l'unité d'affichage (3b) comprend :
un élément d'affichage (3c) indiquant visuellement la pression mesurée ; et
un marquage (3d, 3e) indiquant visuellement une pression cible ou une plage de pression cible.

3. Dispositif de mesure de la concentration en gaz de sulfure (10) selon la revendication 1 ou 2, comprenant en outre :
un orifice de sortie externe (11, 12) ouvert vers une atmosphère ;
une pompe (7) ; et
une valve de commutation (6) configurée pour connecter un orifice de sortie du capteur de gaz de sulfure (5) à un élément sélectionné parmi l'orifice de sortie externe (11, 12) et la pompe (7), l'air expiré étant évacué de l'orifice de sortie du capteur de gaz de sulfure (5).

4. Dispositif de mesure de la concentration en gaz de sulfure (10) selon la revendication 3, comprenant en outre :
un orifice de connexion externe (13) à connecter à une source de gaz de nettoyage fournissant du gaz de nettoyage et configuré pour pouvoir être connecté à l'orifice d'entrée du capteur de gaz de sulfure (5), l'air expiré étant introduit dans l'orifice d'entrée du capteur de gaz de sulfure (5) ; et
un dispositif de commande (15) qui commande le capteur de gaz de sulfure (5), la pompe (7), et la valve de commutation (6) et commande une connexion entre l'orifice de connexion externe (13) et l'orifice d'entrée du capteur de gaz de sulfure (5),
dans lequel, lorsque la concentration en gaz de sulfure dans l'air expiré est mesurée, le dispositif de commande (15) déconnecte l'orifice de connexion externe (13) de l'orifice d'entrée du capteur de gaz de sulfure (5), règle la valve de commutation (6) pour connecter l'orifice de sortie du capteur de gaz de sulfure (5) à l'orifice de sortie externe (13), et amène le capteur de gaz de sulfure (5) à mesurer la concentration en gaz de sulfure dans l'air expiré dans un état, dans lequel la pompe (7) est arrêtée, et
dans lequel, après l'achèvement de la mesure de la concentration en gaz de sulfure dans l'air expiré, le dispositif de commande (15) connecte l'orifice de connexion externe (13) à l'orifice d'entrée du capteur de gaz de sulfure (5) et actionne la pompe (7) dans un état, dans lequel la valve de commutation (6) est réglée pour connecter l'orifice de sortie du capteur de gaz de sulfure (5) à la pompe (7).

5. Procédé de mesure de la concentration en gaz de sulfure en utilisant le dispositif de mesure de la concentration en gaz de sulfure (10) selon l'une quelconque des revendications 1 à 4, le procédé comprenant les étapes consistant à :
placer l'outil de collecte d'air expiré (2) sur un sujet (20) ;
introduire l'air expiré du sujet (20) dans l'orifice d'entrée (4a) du régulateur de pression (4) via l'outil de collecte d'air expiré (2) alors qu'une pression dans l'orifice d'entrée (4a) du régulateur de pression (4) est mesurée et le sujet (20) est averti de la pression mesurée ;
introduire l'air expiré évacué de l'orifice de sortie (4b) du régulateur de pression (4) dans le capteur de gaz de sulfure (5) ; et
mesurer une concentration en gaz de sulfure contenu dans l'air expiré du sujet (20) par le capteur de gaz de sulfure (5).

6. Procédé de mesure de la concentration en gaz de sulfure selon la revendication 5, dans lequel la pression dans l'orifice d'entrée (4a) est mesurée par un dispositif de mesure de pression (3) comprenant une unité d'affichage (3b), et
dans lequel l'unité d'affichage (3b) comprend :
un élément d'affichage (3c) indiquant visuellement la pression mesurée ; et
un marquage (3d, 3e) indiquant visuellement une pression cible ou une plage de pression cible.
